# FASCICULE DE BREVET EUROPEEN

(11) **EP 0 611 566 B1**
(45) Date de publication et mention de la délivrance du brevet: **07.05.1997**
(21) Numéro de dépôt: 94400312.8
(22) Date de dépôt: 14.02.1994
(51) Int. Cl.: A61K 7/06, A61K 7/11

(54) **Laque aqueuse aérosol pour la fixation des cheveux**
Wässriger Aerosolhaarlack für die Haarfixierung
Aqueous aerosol lacque for fixing hair

(30) Priorité: 16.02.1993 FR 9301724
(43) Date de publication de la demande: 24.08.1994
(73) Titulaire: L'OREAL, 75008 Paris (FR)
(72) Inventeur: Lion, Bertrand, F-93190 Livry -Gargan (FR); Mondet, Jean, F-93700 Drancy (FR); Dupuis, Christine, F-75018 Paris (FR)
(74) Mandataire: Stalla-Bourdillon, Bernard

(56) Documents cités:
- EP-A- 0 331 994
- EP-A- 0 406 042
- CA-A- 1 081 617
- FR-A- 2 238 474
- US-A- 4 288 427

## Description

La présente invention a pour objet une composition cosmétique sous forme d'une laque aqueuse aérosol pour la fixation des cheveux contenant en tant que substance filmogène un copolymère essentiellement constitué d'unités d'un acide sulfonique insaturé et d'un N-monoalkylacrylamide ou méthacrylamide et éventuellement d'un autre monomère, les fonctions acides sulfoniques dudit polymère étant neutralisées à l'aide de triéthanolamine, le gaz propulseur de ladite laque étant du diméthyléther.

Pour des raisons écologiques, la tendance actuelle consiste à remplacer systématiquement dans les récipients aérosol pour cheveux, les gaz propulseurs halogénés du type "Fréon", mais la substitution de tels propulseurs par des gaz moins nocifs tels que le diméthyléther (DME) nécessite une profonde modification des formulations. En effet, une telle substitution influe non seulement sur les rapports entre les différents ingrédients, mais également sur la nature même de ceux-ci et tout particulièrement sur celle des substances filmogènes.

Il est en effet bien connu qu'une laque aérosol pour la fixation des cheveux doit satisfaire à un certain nombre de critères et, parmi ceux-ci, un bon pouvoir laquant de la chevelure même en atmosphère à forte teneur en humidité sans que l'on constate un effet de poissage de la chevelure.

Il convient, de plus, que la chevelure présente un aspect naturel, les cheveux étant brillants et souples et qu'ils puissent être peignés sans provoquer un effet de poudrage.

Par ailleurs la viscosité des compositions à pulvériser doit être telle qu'elle puisse permettre une bonne diffusion par l'intermédiaire du dispositif de valve.

Dans le brevet français n° 73.27329, il a été décrit l'utilisation, dans des compositions capillaires et laques aérosol propulsées par des hydrocarbures halogénés, de polymères résultant de la polymérisation de dérivés d'acides sulfoniques éventuellement avec d'autres monomères tels que des dérivés d'acrylates d'alkyle ou des dérivés d'acrylamides.

Ce brevet français, dont l'objet est particulièrement général, ne décrit ni ne suggère toutefois des laques aérosol dont le véhicule est l'eau et le propulseur le diméthyléther.

L'utilisation du diméthyléther en tant qu'agent propulseur de laques à l'eau soulève en effet de nombreuses difficultés quant à la solubilité et à l'homogénéité des compositions.

Après différentes études sur de nombreux copolymères comportant des unités dérivant de la polymérisation d'acides sulfoniques insaturés, on a constaté de façon inattendue et surprenante qu'en vue d'obtenir des laques aérosol ayant à la fois de bonnes propriétés cosmétiques et un bon pouvoir laquant, une sélection très stricte s'imposait, non seulement quant à la nature du copolymère mais également quant à celle de l'agent neutralisant et du taux de neutralisation des fonctions acides sulfoniques du copolymère.

Une telle sélection a en effet permis de mettre en évidence une excellente solubilité dans l'eau des copolymères associée à une bonne compatibilité avec le diméthyléther.

Par ailleurs, on a constaté que les copolymères des laques aérosols selon l'invention devaient en outre répondre à un critère très précis de viscosité en vue d'obtenir des laques aérosol présentant une bonne diffusion.

La présente invention a donc pour objet une laque aérosol pour le maintien et/ou la fixation des cheveux ayant en solution dans l'eau, en tant que substance filmogène, un copolymère constitué essentiellement d'unités répétitives dérivant de la polymérisation
(i) d'au moins un acide sulfonique insaturé en une proportion de 30 à 90 % en poids et ayant la formule générale suivante: 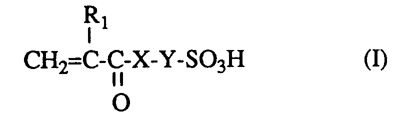 dans laquelle :
   R₁ représente un atome d'hydrogène ou le radical -CH₃,
   X représente -O- ou -NH-, et de préférence -NH-,
   Y représente une chaîne alkylène, linéaire ou ramifiée, ayant de 1 à 6 atomes de carbone, et
(ii) d'au moins un N-monoalkylacrylamide ou méthacrylamide en une proportion de 10 à 70 % en poids et ayant la formule générale suivante: 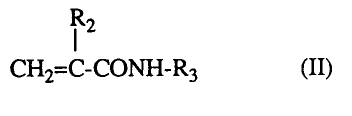 dans laquelle :
   R₂ représente un atome d'hydrogène ou le radical -CH₃ et
   R₃ représente un radical alkyle, linéaire ou ramifié, ayant de 3 à 10 atomes de carbone,
   les fonctions acides sulfoniques dudit polymère étant neutralisées en une proportion de 40 à 70% par de la triéthanolamine, et
   l'agent propulseur de ladite laque aérosol étant du diméthyléther.

Parmi les acides sulfoniques insaturés de formule générale (I), on peut citer notamment l'acide acrylamido-2 méthyl-2 propane sulfonique, la N-acryloyltaurine et la N-méthacryloyltaurine.

Parmi les N-monoalkylacrylamides ou méthacrylamides de formule générale (II), on peut citer notamment le N-tertiobutylacrylamide, le N-tert-hexylacrylamide et le N-tert-octylacrylamide.

Le copolymère selon l'invention résulte de préférence de la copolymérisation d'un acide amidosulfonique (X=-NH-) dont la chaîne alkylène a de 2 à 4 atomes de carbone, et en particulier, de l'acide acrylamido-2 méthyl-2 propane sulfonique, en une proportion de 40 à 70% en poids et d'un N-monoalkylacrylamide et en particulier, du N-tertiobutylacrylamide.

Bien que les copolymères des laques aérosol selon l'invention aient été essentiellement définis comme étant des bipolymères, ceux-ci peuvent également se présenter sous forme de ter-ou tétra-polymères.

Selon cette forme de réalisation, les comonomères susceptibles de constituer les autres unités répétitives du copolymère, peuvent être choisis parmi:
1) les acrylates ou méthacrylates d'alkyle en une proportion de 3 à 40 % en poids et ayant la formule générale suivante: 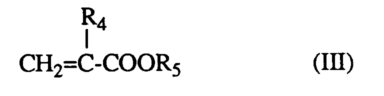 dans laquelle :
   R₄ représente un atome d'hydrogène ou un radical -CH₃, et
   R₅ représente un radical alkyle, linéaire ou ramifié, ayant de 1 à 4 atomes de carbone.

   Parmi les acrylates et méthacrylates d'alkyle de formule (III), on peut notamment citer l'acrylate de méthyle, l'acrylate d'éthyle, et le méthacrylate de butyle.
   Selon une forme de réalisation particulière, on préfère utiliser l'acrylate de méthyle ou l'acrylate d'éthyle en une proportion de 3 à 25 % en poids.
2) les acrylamides et méthacrylamides en une proportion de 3 à 40 % en poids et ayant la formule générale suivante: 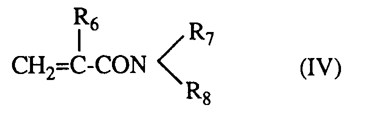 dans laquelle:
   R₆ représente un atome d'hydrogène ou le radical -CH₃, et
   R₇ et R₈, identiques ou différents, représentent un atome d'hydrogène ou un radical alkyle ayant de 1 à 4 atomes de carbone ou R₇ représente un atome d'hydrogène et R₈ représente le radical :

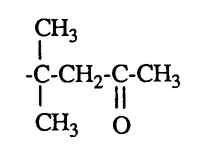

Parmi les acrylamides et méthacrylamides de formule (IV), on peut citer notamment le diméthyl-oxo-3-butyl-acrylamide, le N,N-diméthylacrylamide et le N,N-diéthylacrylamide mais de préférence le diméthyl-oxo-3-butyl-acrylamide en une proportion de 3 à 25 %.

Parmi les copolymères particulièrement préférés selon l'invention, on peut notamment mentionner ceux comportant des unités répétitives dérivant de la copolymérisation de:
- Acide acrylamido-2 méthyl-2 propane sulfonique (62%)/N-tertiobutylacrylamide (38%),
- Acide acrylamido-2 méthyl-2 propane sulfonique (40%)/N-tertiobutylacrylamide (20%)/acrylate d'éthyle (15%)/diméthyl-oxo-3-butylacrylamide (25%),
- Acide acrylamido-2 méthyl-2 propane sulfonique (60%)/N-tertiobutylacrylamide (20%)/diméthyl-oxo-3-butylacrylamide (20%),
- Acide acrylamido-2 méthyl-2 propane sulfonique (60%)/N-tertiobutylacrylamide (25%)/acrylate d'éthyle (15%), et
- Acide acrylamido-2 méthyl-2 propane sulfonique (60%)/N-tertiobutylacrylamide (25%)/acrylate de méthyle (15%).

Comme ceci a été indiqué précédemment, les bonnes propriétés cosmétiques des laques aérosol selon l'invention ne sont pas dues uniquement à la nature du copolymère mais également à celle de l'agent neutralisant utilisé et au taux de neutralisation des fonctions acides sulfoniques.

Les études qui ont été réalisées, en particulier sur le copolymère acide acrylamido-2 méthyl-2 propane sulfonique (62%)/N-tertiobutylacrylamide (38%), ont montré que lorsqu'il est neutralisé à 50 ou 100 % par l'amino-2 méthyl-2 propanol, on obtient une laque ayant un faible pouvoir laquant qui ne peut être amélioré par l'addition d'un plastifiant classique dans la mesure où l'on constate alors certains effets secondaires tels qu'un effet de poissage au toucher par augmentation de l'hygroscopicité.

Parmi les différentes alcanolamines, seule la triéthanolamine s'est avérée satisfaisante en tant qu'agent neutralisant des fonctions acides sulfoniques dans une proportion de 40 à 70% de celles-ci.

La viscosité des copolymères neutralisés à 50 % par la triéthanolamine, en solution aqueuse à 5 % MA et mesurée au Drage Mobile 2 à 25°C est de préférence inférieure à 0,1 Pa.s (100 cps).

Si la viscosité du copolymère était supérieure, on ne pourrait alors obtenir une bonne diffusion du fait des possibilités d'obstruction de la valve.

La viscosité étant directement fonction du poids moléculaire des copolymères, celui-ci est, pour les copolymères des laques selon l'invention, généralement compris entre 10 000 et 500 000 déterminé selon la méthode de chromatographie d'exclusion stérique.

Dans les laques aérosol selon l'invention, le copolymère tel que défini ci-dessus est généralement présent en une proportion comprise entre 2 et 25% en poids par rapport au poids total de la laque, et de préférence entre 5 et 20%.

Bien que la neutralisation du copolymère puisse être réalisée préalablement à son utilisation, celle-ci est de préférence effectuée au sein même de la composition lors de sa formation.

Le véhicule, à savoir l'eau, est généralement présent en une proportion comprise entre 63 et 88% en poids par rapport au poids total de la laque, et de préférence entre 63 et 75%.

L'agent propulseur, à savoir le diméthyléther, est généralement présent en une proportion comprise entre 10 et 35% en poids par rapport au poids total de la laque, ce qui permet de conduire à une solution homogène.

Les laques aérosol selon l'invention peuvent également contenir d'autres ingrédients cosmétiques conventionnels tels que des agents adoucissants, des parfums, des silicones, des filtres solaires, des colorants, des conservateurs, des agents anti-mousse, des vitamines ainsi que des protéines.

Selon l'invention, il est souhaitable que, dans le récipient aérosol, la pression de vapeur soit comprise entre environ 2,5 et 5 bars à 25°C.

Le récipient aérosol dans lequel la composition est conditionnée peut être du type classique ou être éventuellement équipé d'une prise de gaz additionnelle en vue d'obtenir une pulvérisation de qualité plus fine.

Les copolymères de la composition aérosol selon l'invention sont, pour la plupart, connus et peuvent être obtenus par les méthodes classiques de polymérisation, notamment en solution dans un solvant.

Parmi les initiateurs de polymérisation pouvant être utilisés dans le procédé de polymérisation, on peut en particulier citer: le peroxyde de benzoyle, l'azobisisobutyronitrile, l'éthyl-2 perhexanoate de tertiobutyle, le perpivalate de tertiobutyle et le peroxydicarbonate de bis (4-tert-butylcyclohexyle), ces initiateurs étant utilisés soit seuls soit en mélange.

La quantité d'initiateur est généralement comprise entre 0,1 et 6 % en poids par rapport au poids total des monomères à polymériser. La réaction de polymérisation est de préférence effectuée à une température comprise entre 45 et 100°C, et plus particulièrement à la température de reflux du mélange réactionnel.

Le temps de réaction est de préférence compris entre 6 et 24 heures.

On va maintenant donner à titre d'illustration plusieurs exemples de préparation des copolymères ainsi que des exemples de laques aérosol.

### EXEMPLES DE PREPARATION DES COPOLYMERES

### EXEMPLE 1 : Copolymère acide acrylamido-2 méthyl-2 propane sulfonique (AMPS) 62% en poids - N-tertiobutylacrylamide (NTBA) 38 % en poids

Dans un réacteur de deux litres, on introduit successivement 38 g de N-tertiobutyl acrylamide (NTBA) 0,5 g d'azobisisobutyronitrile (AIBN) et 227,5 g d'éthanol et on agite jusqu'à dissolution. Parallèlement, on dissous à part 62 g d'AMPS dans 122,5 g d'eau permutée. La solution aqueuse d'AMPS est versée dans le réacteur en maintenant l'agitation avec barbotage d'azote. Après mélange, on chauffe sous agitation et barbotage d'azote à 70°C en maintenant ces conditions pendant 5 heures.

Au bout de ce temps et retour à température ambiante, on concentre la solution à l'évaporateur rotatif. On purifie par dialyse en utilisant des membranes éliminant les copolymères de PM<6000.

Rendement obtenu : 85 %

Indice d'acide sur le produit séché par lyophilisation : 160 mgKOH/g de produit)

### EXEMPLES 2 à 5 :

En opérant essentiellement comme à l'exemple 1, on a préparé les copolymères du Tableau I.

On procède tout d'abord à la dissolution de l'AMPS dans l'eau puis l'on verse la solution aqueuse obtenue dans le réacteur contenant le reste des monomères, l'éthanol et l'initiateur (AIBN).

Après polymérisation à 70°C pendant 18 heures, on concentre la solution de polymérisation à l'évaporateur rotatif pour éliminer l'éthanol. Après trois jours de dialyse sur membranes éliminant les copolymères de PM d'environ 6000, on procède à une lyophilisation.

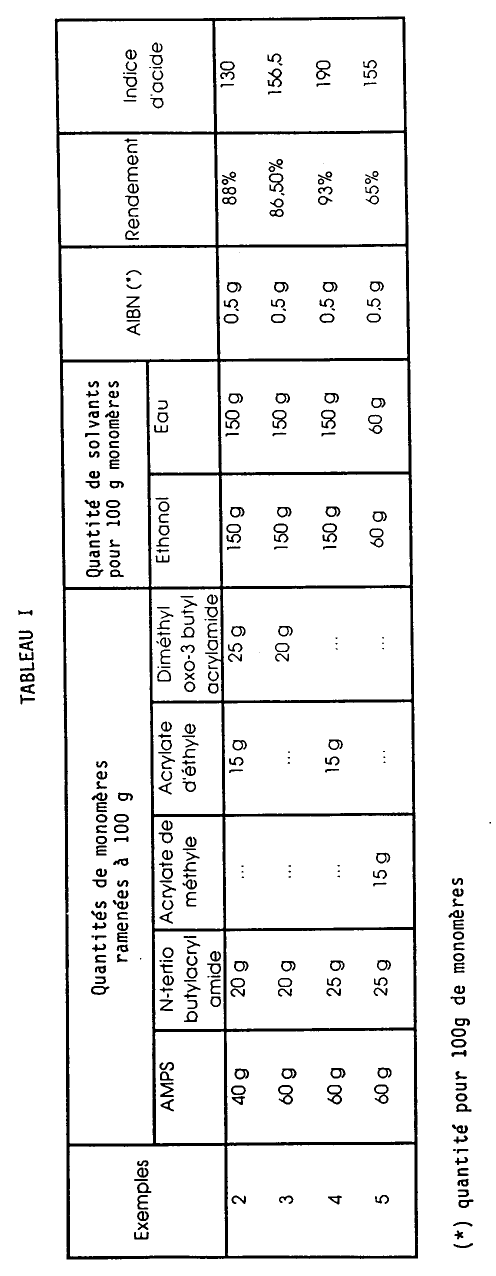

### EXEMPLES DE LAQUES AEROSOL

### EXEMPLE A :

On prépare une laque aérosol pour cheveux en conditionnant, dans un récipient aérosol approprié, 8,4 g du copolymère de l'exemple 1 que l'on neutralise à 60% (d'après l'indice d'acide) par addition de triéthanolamine, un parfum, un conservateur en quantité suffisante, et la quantité suffisante d'eau pour compléter à 70 g.

On introduit ensuite selon les techniques conventionnelles, 30 g de diméthyléther et on procède à la fixation de la valve et à la fermeture hermétique du récipient (pression 4,6 bars).

La valve peut être du type avec prise de gaz additionnelle en vue d'obtenir une pulvérisation de qualité plus fine.

Par application de la laque sur des cheveux naturels ou des cheveux sensibilisés, on constate qu'elle a un excellent pouvoir laquant et ne provoque aucun effet de poissage lors de l'application et après séchage.

### EXEMPLE B :

Selon le même mode opératoire que décrit à l'exemple 1, on a préparé une laque aérosol pour cheveux ayant la composition suivante:

| | |
|---|---|
| - Copolymère de l'exemple 2 | 5,6 g |
| - Triéthanolamine qs pour neutralisation à 50 % | |
| - Parfum qs | |
| - Conservateur qs | |
| - Diméthyléther | 30 g |
| - Eau qsp | 100 g |

### EXEMPLE C :

Selon le même mode opératoire que décrit à l'exemple 1, on a préparé une laque aérosol pour cheveux ayant la composition suivante:

| | |
|---|---|
| - Copolymère de l'exemple 3 | 6,3 g |
| - Triéthanolamine qs pour neutralisation à 50 % | |
| - Parfum qs | |
| - Conservateur qs | |
| - Diméthyléther | 30 g |
| - Eau | 100 g |

### EXEMPLE D :

Selon le même mode opératoire que décrit à l'exemple 1, on a préparé une laque aérosol pour cheveux ayant la composition suivante :

| | |
|---|---|
| - Copolymère de l'exemple 4 | 2,8 g |
| - Triéthanolamine qs pour neutralisation à 70 % | |
| - Parfum qs | |
| - Conservateur qs | |
| - Diméthyléther | 30 g |
| - Eau qsp | 100 g |

### EXEMPLE E :

Selon le même mode opératoire que décrit à l'exemple 1, on a préparé une laque aérosol pour cheveux ayant la composition suivante :

| | |
|---|---|
| - Copolymère de l'exemple 5 | 1,8 g |
| - Triéthanolamine qs pour neutralisation à 60 % | |
| - Parfum qs | |
| - Conservateur qs | |
| - Diméthyléther | 30 g |
| - Eau qsp | 100 g |

## Revendications

1. Laque aqueuse aérosol pour le maintien et/ou la fixation des cheveux, caractérisée par le fait qu'elle contient, en solution dans l'eau, en tant que substance filmogène, un copolymère essentiellement constitué d'unités répétitives dérivant de la polymérisation
(i) d'au moins un acide sulfonique insaturé en une proportion de 30 à 90 % en poids et ayant la formule générale suivante : 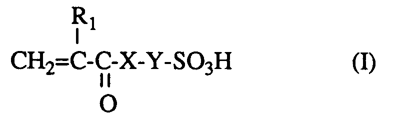 dans laquelle :
R₁ représente un atome d'hydrogène ou le radical CH₃,
X représente -O- ou -NH-, et
Y représente une chaîne alkylène, linéaire ou ramifiée, ayant de 1 à 6 atomes de carbone, et
(ii) d'au moins un N-monoalkylacrylamide ou méthacrylamide en une proportion de 10 à 70 % en poids et ayant la formule générale suivante : 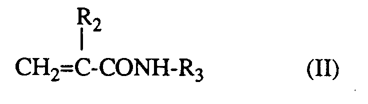 dans laquelle :
R₂ représente un atome d'hydrogène ou le radical -CH₃ et
R₃ représente un radical alkyle, linéaire ou ramifié, ayant de 3 à 10 atomes de carbone,
les fonctions acides sulfoniques dudit copolymère étant neutralisées en une proportion de 40 à 70 % par de la triéthanolamine, et
l'agent propulseur de ladite laque aérosol étant du diméthyléther.

2. Laque aérosol selon la revendication 1, caractérisée par le fait que l'acide sulfonique insaturé de formule (I) est choisi parmi l'acide acrylamido-2 méthyl-2 propane sulfonique, la N-acryloyltaurine et la N-méthacryloyltaurine.

3. Laque aérosol selon la revendication 1, caractérisée par le fait que le N-monoalkylacrylamide ou méthacrylamide de formule (II) est choisi parmi le N-tertiobutyl acrylamide, le N-tert-hexylacrylamide et le N-tert-octylacrylamide.

4. Laque aérosol selon l'une quelconque des revendications précédentes, caractérisée par le fait que le copolymère résulte de la copolymérisation d'un acide amidosulfonique dont la chaîne alkylène a de 2 à 4 atomes de carbone, en une proportion de 40 à 70 % en poids, et d'un N-mono alkylacrylamide.

5. Laque aérosol selon l'une quelconque des revendications précédentes, caractérisée par le fait que le fait que le copolymère comporte en outre des unités répétitives dérivant de la copolymérisation d'un acrylate ou méthacrylate d'alkyle, en une proportion de 3 à 40 % en poids, et ayant la formule générale suivante : 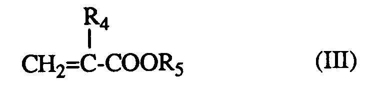 dans laquelle :
R₄ représente un atome d'hydrogène ou un radical -CH₃, et
R₅ représente un radical alkyle, linéaire ou ramifié, ayant de 1 à 4 atomes de carbone.

6. Laque aérosol selon la revendication 5, caractérisée par le fait que l'acrylate ou méthacrylate d'alkyle de formule (III) est choisi parmi l'acrylate de méthyle, l'acrylate d'éthyle et le méthacrylate de butyle.

7. Laque aérosol selon la revendication 5 ou 6, caractérisée par le fait que les unités d'acrylate ou méthacrylate d'alkyle sont présentes en une proportion de 3 à 25 % en poids du polymère.

8. Laque aérosol selon l'une quelconque des revendications précédentes, caractérisée par le fait que le polymère comporte en outre des unités répétitives dérivant de la polymérisation d'un acrylamide ou méthacrylamide en une proportion de 3 à 40 % en poids et ayant la formule générale suivante : 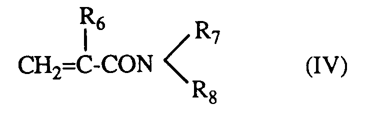 dans laquelle :
R₆ représente un atome d'hydrogène ou le radical -CH₃, et
R₇ et R₈, identiques ou différents, représentent un atome d'hydrogène ou un radical alkyle ayant de 1 à 4 atomes de carbone ou R₇ représente un atome d'hydrogène et R₈ représente le radical :

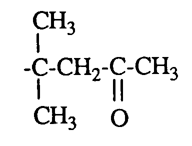

9. Laque aérosol selon la revendication 8, caractérisée par le fait que l'acrylamide ou méthacrylamide de formule (IV) est choisi parmi le diméthyl-oxo-3-butylacrylamide, le N,N-diméthylacrylamide et le N,N-diéthylacrylamide.

10. Laque aérosol selon la revendication 8 ou 9, caractérisée par le fait que les unités d'acrylamide ou méthacrylamide sont présentes en une proportion de 3 à 25 % en poids du copolymère.

11. Laque aérosol selon l'une quelconque des revendications précédentes, caractérisée par le fait que le copolymère comporte des unités répétitives dérivant de la copolymérisation de:
- Acide acrylamido-2 méthyl-2 propane sulfonique (62%)/N-tertiobutylacrylamide (38%),
- Acide acrylamido-2 méthyl-2 propane sulfonique (40%)/N-tertiobutylacrylamide (20%)/acrylate d'éthyle (15%)/diméthyl-oxo-3-butylacrylamide (25%),
- Acide acrylamido-2 méthyl-2 propane sulfonique (60%)/N-tertiobutylacrylamide (20%)/diméthyl-oxo-3-butylacrylamide (20%),
- Acide acrylamido-2 méthyl-2 propane sulfonique (60%)/N-tertiobutylacrylamide (25%)/acrylate d'éthyle (15%), et
- Acide acrylamido-2 méthyl-2 propane sulfonique (60%)/N-tertiobutyl acrylamide (25%)/acrylate de méthyle (15%).

12. Laque aérosol selon l'une quelconque des revendications précédentes, caractérisée par le fait que la viscosité du copolymère neutralisé à 50 % par la triéthanolamine, en solution aqueuse à 5 % MA et mesurée au Drage Mobile 2 à 25°C est inférieure à 0,1 Pa.s (100 cps).

13. Laque aérosol selon l'une quelconque des revendications précédentes, caractérisée par le fait que le copolymère présente un poids moléculaire compris entre 10.000 et 500.000 mesuré selon la méthode de chromatographie d'exclusion stérique.

14. Laque aérosol selon l'une quelconque des revendications précédentes, caractérisée par le fait que le copolymère est présent en une proportion comprise entre 2 et 25 % en poids par rapport au poids total de la laque, et de préférence entre 5 et 20 %.

15. Laque aérosol selon l'une quelconque des revendications précédentes, caractérisée par le fait que l'eau est présente en une proportion comprise entre 63 et 88 % en poids par rapport au poids total de la laque, et de préférence entre 63 et 75 %.

16. Laque aérosol selon l'une quelconque des revendications précédentes, caractérisée par le fait que le diméthyléther est présent en une proportion comprise entre 10 et 35 % en poids par rapport au poids total de la laque aérosol.

17. Laque aérosol selon l'une quelconque des revendications précédentes, caractérisée par le fait qu'elle contient en outre au moins un ingrédient cosmétique conventionnel choisi parmi les agents adoucissants, les parfums, les silicones, les filtres solaires, les colorants, les conservateurs, les agents anti-mousse, les vitamines et les protéines.

## Claims

1. Aqueous aerosol lacquer for maintaining and/or setting hair, characterized in that it contains, in solution in water, as film-forming substance, a copolymer essentially consisting of repeat units derived from the polymerization
(i) of at least one unsaturated sulphonic acid in a proportion of 30 to 90 % by weight and having the following general formula: 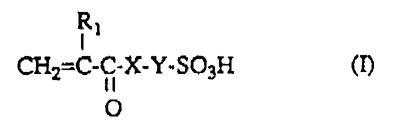 in which:
R₁ denotes a hydrogen atom or the CH₃ radical,
X denotes -O- or -NH-, and
Y denotes a linear or branched alkylene chain containing from 1 to 6 carbon atoms, and
(ii) of at least one N-monoalkylacrylamide or methacrylamide in a proportion of 10 to 70 % by weight and having the following general formula: 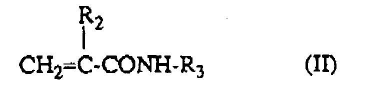 in which:
R₂ denotes a hydrogen atom or the -CH₃ radical and
R₃ denotes a linear or branched alkyl radical containing from 3 to 10 carbon atoms,
the sulphonic acid functional groups of the said copolymer being neutralized in a proportion of 40 to 70 % with triethanolamine, and
the propellent agent of the said aerosol lacquer being dimethyl ether.

2. Aerosol lacquer according to Claim 1, characterized in that the unsaturated sulphonic acid of formula (I) is chosen from 2-acrylamido-2-methylpropanesulphonic acid, N-acryloyltaurine and N-methacryloyltaurine.

3. Aerosol lacquer according to Claim 1, characterized in that the N-monoalkylacrylamide or methacrylamide of formula (II) is chosen from N-tert-butylacrylamide, N-tert-hexylacrylamide and N-tert-octylacrylamide.

4. Aerosol lacquer according to any one of the preceding claims, characterized in that the copolymer results from the copolymerization of an amidosulphonic acid in which the alkylene chain has from 2 to 4 carbon atoms, in a proportion of 40 to 70 % by weight, and of an N-monoalkylacrylamide.

5. Aerosol lacquer according to any one of the preceding claims, characterized in that the copolymer additionally contains repeat units derived from the copolymerization of an alkyl acrylate or methacrylate in a proportion of 3 to 40 % by weight and having the following general formula: 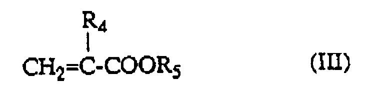 in which:
R₄ denotes a hydrogen atom or a -CH₃ radical, and
R₅ denotes a linear or branched alkyl radical which has from 1 to 4 carbon atoms.

6. Aerosol lacquer according to Claim 5, characterized in that the alkyl acrylate or methacrylate of formula (III) is chosen from methyl acrylate, ethyl acrylate and butyl methacrylate.

7. Aerosol lacquer according to Claim 5 or 6, characterized in that the alkyl acrylate or methacrylate units are present in a proportion of 3 to 25 % by weight of the polymer.

8. Aerosol lacquer according to any one of the preceding claims, characterized in that the polymer additionally contains repeat units derived from the polymerization of an acrylamide or methacrylamide in a proportion of 3 to 40 % by weight and having the following general formula: 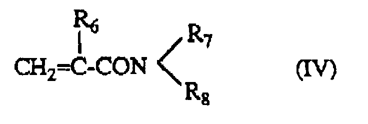 in which:
R₆ denotes a hydrogen atom or the -CH₃ radical, and
R₇ and R₈, which are identical or different, denote a hydrogen atom or an alkyl radical which has from 1 to 4 carbon atoms or R₇ denotes a hydrogen atom and R₈ denotes the radical:

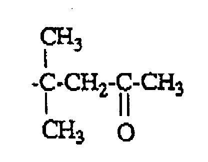

9. Aerosol lacquer according to Claim 8, characterized in that the acrylamide or methacrylamide of formula (IV) is chosen from dimethyl-3-oxobutylacrylamide, N,N-dimethylacrylamide and N,N-diethylacrylamide.

10. Aerosol lacquer according to Claim 8 or 9, characterized in that the acrylamide or methacrylamide units are present in a proportion of 3 to 25 % by weight of the copolymer.

11. Aerosol lacquer according to any one of the preceding claims, characterized in that the copolymer contains repeat units derived from the copolymerization of:
- 2-acrylamido-2-methylpropanesulphonic acid (62 %)/N-tert-butylacrylamide (38 %),
- 2-acrylamido-2-methylpropanesulphonic acid (40 %)/N-tert-butylacrylamide (20 %)/ethyl acrylate (15 %)/dimethyl-3-oxobutylacrylamide (25 %),
- 2-acrylamido-2-methylpropanesulphonic acid (60 %)/N-tert-butylacrylamide (20 %)/dimethyl-3-oxobutylacrylamide (20 %),
- 2-acrylamido-2-methylpropanesulphonic acid (60 %)/N-tert-butylacrylamide (25 %)/ethyl acrylate (15 %), and
- 2-acrylamido-2-methylpropanesulphonic acid (60 %)/N-tert-butylacrylamide (25 %)/methyl acrylate (15 %).

12. Aerosol lacquer according to any one of the preceding claims, characterized in that the viscosity of the copolymer 50% neutralized with triethanolamine, in aqueous solution containing 5 % AS and measured with the Drage 2 rotor at 25°C is lower that 0.1 Pa s (100 cps).

13. Aerosol lacquer according to any one of the preceding claims, characterized in that the copolymer has a molecular weight of between 10,000 and 500,000 measured by the steric exclusion chromatography method.

14. Aerosol lacquer according to any one of the preceding claims, characterized in that the copolymer is present in a proportion of between 2 and 25 % by weight relative to the total weight of the lacquer, and preferably between 5 and 20 %.

15. Aerosol lacquer according to any one of the preceding claims, characterized in that the water is present in a proportion of between 63 and 88 % by weight relative to the total weight of the lacquer, and preferably between 63 and 75 %.

16. Aerosol lacquer according to any one of the preceding claims, characterized in that dimethyl ether is present in a proportion of between 10 and 35 % by weight relative to the total weight of the aerosol lacquer.

17. Aerosol lacquer according to any one of the preceding claims, characterized in that it additionally contains at least one conventional cosmetic ingredient chosen from softening agents, perfumes, silicones, sunscreens, dyes, stabilizers, antifoam agents, vitamins and proteins.

## Patentansprüche

1. Lackaerosol auf Wasserbasis zum Halten und/oder Fixieren der Haare, dadurch gekennzeichnet, daß er in wäßriger Lösung als filmbildende Substanz ein Copolymer enthält, welches im wesentlichen aus wiederkehrenden Einheiten besteht, aus der Polymerisation von
(i) mindestens einer ungesättigten Sulfonsäure der folgenden allgemeinen Formel in einer Menge von 30 bis 90 Gew.-% 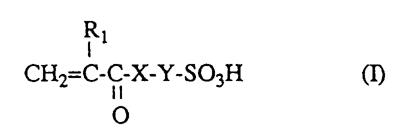 in der
R₁ ein Wasserstoffatom oder eine CH₃-Gruppe,
X -O- oder -NH- und
Y einen geraden oder verzweigten Alkylenrest mit 1 bis 16 Kohlenstoffatomen darstellt, mit
(ii) mindestens einem N-Monoalkylacrylamid oder -methacrylamid der folgenden allgemeinen Formel in einer Menge von 10 bis 70 Gew.-% 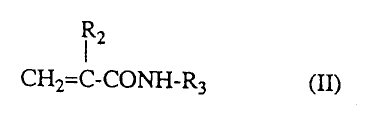 in der
R₂ ein Wasserstoffatom oder eine CH₃-Gruppe und
R₃ einen geraden oder verzweigten Alkylrest mit 3 bis 10 Kohlenstoffatomen darstellt,
wobei die Sulfonsäurefunktionalitäten des Polymeren zu 40 bis 70 % mit Triethanolamin neutralisiert sind und das Triebmittel des Aerosollackes Dimethylether ist.

2. Lackaerosol gemäß Anspruch 1, dadurch gekennzeichnet, daß die ungesättigte Sulfonsäure der Formel (I) unter 2-Acrylamido-2-methylpropansulfonsäure, dem N-Acryloyltaurin und dem N-Methacryloyltaurin ausgewählt ist.

3. Lackaerosol gemäh Anspruch 1, dadurch gekennzeichnet, daß das N-Monoalkylacrylamid oder -methacrylamid der Formel (II) aus N-tert.-Butylacrylamid, N-tert.-Hexylacrylamid und N-tert.-Octylacrylamid ausgewählt ist.

4. Lackaerosol gemäß einem der vorstehenden Ansprüche, dadurch gekennzeichnet, daß das Copolymer aus der Copolymerisation einer Amidosulfonsäure mit einer Alkylkette mit 2 bis 4 Kohlenstoffatomen in einer Menge von 40 bis 70 Gew.-% und einem N-Monoalkylacrylamid stammt.

5. Lackaerosol gemäß einem der vorstehenden Ansprüche, dadurch gekennzeichnet, daß das Copolymer außerdem wiederkehrende Einheiten aufweist, die aus der Copolymerisation eines Alkylacrylats oder -methacrylats mit folgender allgemeiner Formel in einer Menge von 3 bis 40 Gew.-% stammen: 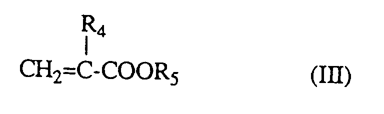 in der
R₄ ein Wasserstoffatom oder eine CH₃-Gruppe und
R₅ einen geraden oder verzweigten Alkylrest mit 1 bis 4 Kohlenstoffatomen darstellt.

6. Lackaerosol gemäß Anspruch 5, dadurch gekennzeichnet, daß das Alkylacrylat oder - methacrylat der Formel (III) aus Methylacrylat, Ethylacrylat und Butylmethacrylat ausgewählt ist.

7. Lackaerosol gemäß Anspruch 5 oder 6, dadurch gekennzeichnet, daß die Alkylacrylat- oder -methacrylateinheiten 3 bis 25 Gew.-% des Polymeren ausmachen.

8. Lackaerosol gemäß einem der vorstehenden Ansprüche, dadurch gekennzeichnet, daß das Copolymer außerdem wiederkehrende Einheiten aufweist, die aus der Copolymerisation eines Alkylacrylats oder -methacrylats mit folgender allgemeiner Formel in einer Menge von 3 bis 40 Gew.-% stammen: 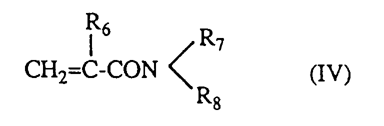 in der
R₆ ein Wasserstoffatom oder eine CH₃-Gruppe sowie
R₇ und R₈, die gleich oder verschieden sind, ein Wasserstoffatom oder einen Alkylrest mit 1 bis 4 Kohlenstoffatomen darstellen oder R₇ Wasserstoff ist und R₈ einen Rest 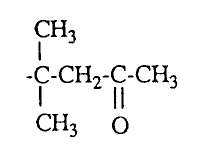 darstellt.

9. Lackaerosol gemäß Anspruch 8, dadurch gekennzeichnet, daß das Acrylamid oder Methacrylamid der Formel (IV) aus Dimethyl-3-oxobutylacrylamid, N,N-Dimethylacrylamid und N,N-Diethylacrylamid ausgewählt ist.

10. Lackaerosol gemäß Anspruch 8 oder 9, dadurch gekennzeichnet, daß die Arcyl- oder Methacryleinheiten 3 bis 25 Gew.-% des Polymeren ausmachen.

11. Lackaerosol gemäß einem der vorstehenden Ansprüche, dadurch gekennzeichnet, daß das Copolymer aus der Polymerisation von
- 2-Acrylamido-2-methylpropansulfonsäure (62%)/N-tert.-Butylacrylamid (38%)
- 2-Acrylamido-2-methylpropansulfonsäure (40%)/N-tert.-Butylacrylamid (20%)/Ethylacrylat (15%)/Dimethyl-3-oxobutylacrylamid (25%)
- 2-Acrylamido-2-methylpropansulfonsäure (60%)/N-tert.-Butylacrylamid (20%)/Dimethyl-3-oxobutynacrylamid (20%)
- 2-Acrylamido-2-methylpropansulfonsäure (60%)/N-tert.-Butylacrylamid (25%)/Ethylacrylat (15%) und
- 2-Acrylamido-2-methylpropansulfonsäure (60%)/N-tert.-Butylacrylamid (25%)/Methylacrylat (15%)
stammende wiederkehrende Einheiten aufweist.

12. Lackaerosol gemäß einem der vorstehenden Ansprüche, dadurch gekennzeichnet, daß die Viskosität des zu 50 % mit Triethanolamin neutralisierten Copolymeren in wäßriger Lösung mit 5 % MA, gemessen mit Hilfe eines Drage Mobile bei 2 bis 25 °C unterhalb von 0,1 Pa · s (100 cps) liegt.

13. Lackaerosol gemäß einem der vorstehenden Ansprüche, dadurch gekennzeichnet, daß das Copolymer ein über sterische Exclusionschromatographie gemessenes Molekulargewicht zwischen 10.000 und 500.000 aufweist.

14. Lackaerosol gemäß einem der vorstehenden Ansprüche, dadurch gekennzeichnet, daß das Copolymer in einer Menge von 2 bis 25 Gew.-%, bezogen auf das Gesamtgewicht des Lackes, vorzugsweise 5 bis 20 Gew.-% vorliegt.

15. Lackaerosol gemäß einem der vorstehenden Ansprüche, dadurch gekennzeichnet, daß das Wasser in einer Menge von 63 bis 88 Gew.-%, bezogen auf das Gesamtgewicht des Lackes, vorzugsweise 63 bis 75 Gew.-% vorliegt.

16. Lackaerosol gemäß einem der vorstehenden Ansprüche, dadurch gekennzeichnet, daß der Dimethylether in einer Menge von 10 bis 35 Gew.-%, bezogen auf das Gesamtgewicht des Lackaerosols, vorliegt.

17. Lackaerosol gemäß einem der vorstehenden Ansprüche, dadurch gekennzeichnet, daß es zusätzlich mindestens einen herkömmlichen kosmetischen Bestandteil enthält, der ausgewählt ist aus Weichmachern, Parfümen, Silikonen, UV-Filtern, Farbstoffen, Konservierungsmitteln, Schaumbremsern, Vitaminen und Proteinen.
